(19) **Europäisches Patentamt / European Patent Office / Office européen des brevets**

(11) **EP 1 854 870 B1**

(12) **EUROPEAN PATENT SPECIFICATION**

(45) Date of publication and mention
of the grant of the patent:
**04.08.2010 Bulletin 2010/31**

(21) Application number: **06712396.8**

(22) Date of filing: **26.01.2006**

(51) Int Cl.:
*C12M 1/00* (2006.01)     *B82B 3/00* (2006.01)
*C12M 1/42* (2006.01)     *C12N 13/00* (2006.01)
*C12N 15/02* (2006.01)

(86) International application number:
**PCT/JP2006/301214**

(87) International publication number:
**WO 2006/087890 (24.08.2006 Gazette 2006/34)**

(54) **ELECTRODE PAIR NONCONTACT MANIPULATION DEVICE AND MANIPULATION METHOD**

KONTAKTLOSE ELEKTRODENPAARMANIPULATIONSVORRICHTUNG UND
MANIPULATIONSVERFAHREN

DISPOSITIF DE MANIPULATION SANS CONTACT AVEC UNE PAIRE D'ELECTRODES ET
PROCÉDÉ DE MANIPULATION

(84) Designated Contracting States:
**DE FR GB**

(30) Priority: **15.02.2005 JP 2005038212**

(43) Date of publication of application:
**14.11.2007 Bulletin 2007/46**

(73) Proprietor: **KOCHI UNIVERSITY OF
TECHNOLOGY
Kami-shi,
Kochi 782-8502 (JP)**

(72) Inventors:
• **FURUSAWA, Hiroshi
Kochi 781-5103 (JP)**
• **KAGEYAMA, Daisuke
Takashima-shi
520-1501 Shiga (JP)**

• **NAGASAKA, Wako
Makinohara-shi
421-0414 Shizuoka (JP)**

(74) Representative: **Babeluk, Michael
Patentanwalt
Mariahilfer Gürtel 39/17
1150 Wien (AT)**

(56) References cited:
| | |
|---|---|
| WO-A-00/17322 | WO-A-03/104448 |
| JP-A- 4 349 889 | JP-A- 5 137 576 |
| JP-A- 04 349 889 | JP-A- 05 137 576 |
| JP-A- 61 209 592 | JP-A- 2000 241 714 |
| JP-A- 2000 241 714 | JP-A- 2004 041 023 |
| JP-A- 2004 041 023 | JP-A- 2004 148 026 |
| US-A1- 2003 157 708 | US-A1- 2003 199 050 |

**Description**

BACKGROUND OF THE INVENTION

Field of Invention

The present invention relates to a manipulation device and the manipulation method which move a microparticle contained in the suspension liquid to a specific' location and to perform manipulations such as selection, binding fusion and more particularly it concerns a manipulation device and the manipulation method which may perform the manipulation without bringing an electrode into contact with a particle.

Description of the Background Art

Electric field induced fusion and microelectrode techniques have been known as methods to create a heterokaryon by the fusion of a cell A and a cell B having genetically different nuclei.

In electric field induced fusion, cell suspension liquid containing two types of cells is placed between a pair of parallel electrodes, and an AC voltage is applied to the space between the pair of parallel electrodes. In this way, pearl chain (where cells are aligned) is created. A DC pulse is applied to the pearl chain to fuse the cells.

In microelectrode techniques, a microelectrode is used. The microelectrode comprises cylindrical electrode inserted into a glass micro tube, so that the end of the cylindrical electrode is exposed, where the inner wall of the glass tube tightly contacts with the outer wall of the electrode. An AC voltage is applied to the microelectrodes, to attract the cells in the cell suspension liquid and to make adherent to the end of the microelectrode by dielectrophoresis. Then, an electrical pulse is applied to the cells to create a heterokaryon.

In electric field induced fusion, it is difficult to fuse the selected cells. For example, it is not possible to select two types of cells, where a cell A and a cell B have genetically different cell nuclei, such that the cells are fuse to create a new heterokaryon A·B. Furthermore it is not possible to control the number of cells to be fused. For example, three cells may be fused even though one wants to fuse just two cells.

Microelectrode techniques require the selective detachment of the heterokaryon adhered to the end of the micro tube. It requires good experiences to selectively detach the heterokaryon from the microelectrode without damaging. Japanese patent publication H5-137576 discloses a way to solve the problems associated with the above mentioned methods.

Fig. 13 shows a cell fusion device disclosed in Japanese patent publication H5-137576.

The cell fusion device disclosed in Japanese patent publication H5-137576 is equipped with a needle electrode (N) and a plate electrode (P). The space between the end of the needle electrode (N) and the plate electrode (P) is filled with cell suspension liquid (S) which contains cells (C). An insulation cover (I) is placed to cover the end of the needle electrode (N). Between the insulation cover (I) and the needle electrode (N) is formed some space. The end of the insulation cover (I) has a through-hole (A) through which the suspension liquid (S) reaches the end of the needle electrode (N) placed in the insulation cover (I). The size of the through-hole (A) is made smaller than the cell (C) contained in the suspension liquid (S). The axis of the needle electrode (N) is subsubtantially vertical with respect to the plate electrode (P). When an AC voltage is applied to the needle electrode (N) and the plate electrode (P), an electric field is induced across the layer of suspension liquid (S) between the needle electrode (N) and the plate electrode (P). Since the electrode with a sharp end directs to the electrode with a plane surface, the induced electric field is inhomogeneous. The cell (C) is attracted to the vicinity of the through-hole (A) by the induced electric field.

By using the cell fusion device with the above mentioned components, the needle electrode (N) and the plate electrode (P) are first put into suspension liquid which contains one type of cells. Then an electric field is induced between the electrodes (N, P) to make one type of the cell adhered to the vicinity of the through-hole (A). After then, the needle electrode (N) and the plate electrode (P) are put into another suspension liquid containing another type of cells. Then another type of cell becomes adhered to the vicinity of the through-hole (A). And then the different type of cells attached to the vicinity of the through-hole (A) are fused. Since the fused cell is on the insulation cover (I), it may be easily detached from the insulation cover (I) by terminating the application of the AC voltage.

another type of cell is made adhered to the vicinity of the through-hole (A). And then the different type of cells attached to the vicinity of the through-hole (A) are fused. Since the fused cell is on the insulation cover (I), it may be easily detached from the insulation cover (I) by terminating the application of the AC voltage.

As described above, the cell fusion device of Japanese patent publication H5-137576 solves the problems related to the conventional electric field induced fusion and microelectrode techniques. It has however such problems as described below. The cell fusion device of Japanese patent publication H5-137576 enables only to attract a cell toward the needle electrode (N). This means it is not able to perform any other manipulation than the attraction. For example, it is not possible to perform a manipulation such as pushing a cell away to a desired direction without any contact or orienting a cell toward a desired direction.

An electroporation device is shown in the WO 03/104448 A. There is a working electroporation device wherein the control of the voltage applied to the substrate is obtained by means of the continuous monitoring of the ratio of the current flowing through the substrate and the voltage applied to the substrate. According to another embodiment of WO 03/104448 A, the control of the voltage is obtained by simply monitoring the current. In particular, the device comprises of a signal generator for producing a stimulating signal that is amplified by power amplifier and applied to electrodes through a power switch. The electrodes are coupled with a substrate containing living cells. The application of the electroporating signal to the electrodes causes the creation of an electric field in the substrate; such a filed promotes, realises or enhances the permeabilization of the membranes of the cells rendering possible the introduction of molecules (organic/inorganic) through the membranes of the cells. The device also comprises a measuring signal generator coupled with a measurement block co-operating with electrodes for the determination of the instantaneous values of the current through the substrate, the voltage actually applied to the substrate, and the ratio of the current through the voltage applied to the substrate. In actual use, electrodes are applied to the substrate containing live cells - indirect application of the substance, e.g. by introducing the substance into the circulatory system of the tissue portion forming the substrate and - injecting the substance, e.g. using needlelike electrodes, each having an inner conduit containing the substance to be injected into the tissue portion forming the substrate. The substance may also be injected using needles separate from the electrodes. The substrate can also be treated without the application of a substance when the purpose is to extract from the cells a molecule (organic or inorganic or biopolymeric) contained in or produced by the cells. The minimum has been reached, the resulting curve has a too rapid increase in fact, a too rapid increase after the minimum is a clear indication of irreversible damages to the cells. In case of a detected indication of irreversible damages a corrective action is performed by immediately decreasing the voltage applied thus preventing final damage to the cells.

Consequently, the conventional manipulation devise do not allow manipulation such as pushing a cell into a narrow channel without any contact, and moving a cell as desired in the channel, or orienting a cell toward the desired direction. In order to overcome the above described problems, this invention aims to provide a manipulation device and the method to enable the manipulation of the microparticle which has been difficult by the conventional devices and to enable an easy movement or a rotational movement of the microparticle.

In particular, it is an object of the present invention to create a non-contact manipulation device and a method for manipulating and for moving the microparticles, such as cells, without any contact. microparticles, at least a pair of electrodes having the distal ends inserted into the suspension liquid layer, and a power supply for supplying an AC voltage to the electrodes, wherein the at lease a pair of electrodes configured to move independently of each other, and wherein an electric field is generated substantially parallel to the surface of the suspension liquid layer between the at least a pair of electrodes.

The invention described in claim 2 is a non-contact manipulation device with an electrode pair comprising a base, a suspension liquid layer disposed on the base, at least a pair of electrodes having the distal ends inserted into the suspension liquid layer, and a power supply for supplying an AC voltage to the electrodes, wherein the at least a pair of electrodes is configured to move independently of each other, the suspension liquid layer comprises a first liquid and a second liquid having a dielectric constant different from the first liquid, the second liquid occupies a microscopic region of the suspension liquid layer, an electric field is generated substantially parallel to the surface of the suspension liquid layer between the at least a pair of electrodes.

The invention described in claim 3 is the non-contact manipulation device with an electrode pair according to claim 1 or 2, wherein the power supply is configured to modulate the frequency of the AC voltage.

The invention described in claim 4 is the non-contact manipulation device with an electrode pair according to claim 1 or 2, wherein the power supply is configured to modulate the amplitude of the AC voltage.

The invention described in claim 5 is the non-contact manipulation device with an electrode pair according to claim 1 or 2, wherein the power supply is configured to modulate the waveform of the AC voltage.

The invention described in claim 6 is the non-contact manipulation device with an electrode pair according to claim 1 or 2, wherein the power supply is configured to apply a DC voltage.

The invention described in claim 7 is the non-contact manipulation device with an electrode pair according to claim 1 or 2, wherein the base is placed in observable area through a microscope to allow the microparticles to be observed.

The invention described in claim 8 is the non-contact manipulation device with an electrode pair according to claim 1 or 2, wherein the at least a pair of electrodes is multi-pairs of electrodes, and a phase difference of the AC voltage between one pair of electrodes and the other pair of electrodes.

The invention described in claim 9 is a manipulation method of the non-contact manipulation device comprising selecting a microparticle in the suspension liquid layer, placing at least a pair of electrodes such that the selected microparticle is placed between the electrodes and is not in contact with the electrodes, applying voltage to the electrodes, adjusting the frequency of the voltage, and moving the electrodes such that the at least a pair of electrodes is configured to move independently of each other. The invention described in claim 10 is the non-contact manipulation method with an electrode pair according to claim 9, wherein the suspension liquid layer is placed on a first and a second operation area, which are connected through a narrow channel.

The invention described in claim 11 is a non-contact manipulation method with an electrode pair according to claim 10, wherein the microparticle is ellipsoidal, and the width of the narrow channel is narrower than a long axis of the particle and wider than a short axis of the particle.

The invention described in claim 12 is a non-contact manipulation method with an electrode pair according to claim 9, wherein the at least a pair of electrodes is multi-pairs of electrodes, and the step of adjusting the frequency of the voltage further comprising modification of a phase difference of the AC voltage between one pair of electrodes and the other pair of electrodes.

The invention described in claim 13 is a non-contact manipulation method with an electrode pair according to claim 9, wherein the at least a pair of electrodes is multi-pairs of electrodes, and the step of moving the electrodes further comprising modification of an angle formed between an electric field created by a pair of electrodes and an electric field created by other pair of electrodes of the multi pairs of electrodes.

The invention described in claim 14 is a non-contact manipulation method with an electrode pair according to claim 9, wherein the microparticle is a cell, and the step of moving the electrodes comprises degradation of the suspension liquid layer environment after placing the cell next to other cells.

The invention described in claim 15 is a non-contact manipulation method with an electrode pair according to claim 9, wherein the microparticle is a cell, and the step of moving the electrodes comprises, placing the cell to form a cell population which is made up with cells lined next to each other, and applying a DC voltage to the distal of the cell population.

The invention described in claim 16 is a non-contact manipulation method with an electrode pair according to claim 14 or 15, wherein the step of moving the electrodes comprising adjustment of the cell orientation.

The invention described in claim 17 is a non-contact manipulation method with an electrode pair comprising selecting a cell in the suspension liquid layer, placing at least one pair of electrodes such that the selected cell is placed between the electrodes and is not in contact with the cell, applying voltage to the electrodes, to destroy external structures of the cell, and to release internal organelles of the cell, applying the voltage to the electrodes, adjusting the frequency of the voltage, and moving the electrodes, wherein the at least a pair of electrodes configured to move independently of each other.

The invention described in claim 18 is a non-contact manipulation method with an electrode pair comprising dropping a second liquid into a liquid layer comprised of the first liquid, the second liquid having a dielectric constant different from the first liquid, selecting one of areas formed in the liquid layer which is comprised of the second liquid, placing at least one pair of electrodes such that the selected area is placed between the electrodes and not to touch the area, applying voltage to the electrodes, adjusting the frequency of the voltage, and moving the electrodes, wherein the at least a pair of electrodes is configured to move independently of each other.

Effect of the Invention

According to the invention of claim 1, by moving at least a pair of electrodes independently of each other, it is possible to move a particular microparticle between the electrodes to a desired position, or to rotate it to a desired orientation.

According to the invention of claim 2, by moving at least a pair of electrodes independently of each other, it is possible to move a particular microscopic region between the electrodes to a desired position, or to rotate it to a desired orientation.

According to the invention of claim 3, it is possible to control the moving direction of the microparticle or the microscopic region by adjusting the frequency depending on the type of the microparticle or the microscopic region.

According to the invention of claim 4, it is possible to control the velocity of the microparticle or the microscopic region.

According to the invention of claim 5, it is possible to control the change in the velocity of the microparticle or the microscopic region.

According to the invention of claim 6, with behavior of the microparticle or the microscopic region observed, it is possible to adjust the movement of the microparticle or the microscopic region.

According to the invention of claim 7, it is possible to move the microparticle to a particular direction. Alternatively, when the microparticle is a cell, it is possible to manipulate a cell to fuse.

According to the invention of claim 8, it is possible to provide a particle with more complicated movement and with more precise positional control.

According to the invention of claim 9, a microparticle may be moved toward a desired direction. Alternatively, a cell may be oriented to a desired direction.

According to the invention of claim 10, it is possible to easily sort microparticles.

According to the invention of claim 11, it is possible to prevent unwanted mixture of microparticles in the first and the second operation areas, to easily perform more effective and precise selection of a particle.

According to the invention of claim 12 and claim 13, it is also possible to perform precise positional manipulation of a particle as well as more complicated movement of a particle.

According to the invention of claim 14 and claim 15, it is possible to perform cell fusion on a desired position. According to the invention of claim 16, it is possible to perform a cell fusion while a cell is oriented in the desired direction.

According to the invention of claim 17, it is possible to effectively collect cellular organelles. According to the invention of claim 18, it is possible to easily move microscopic region.

Examples

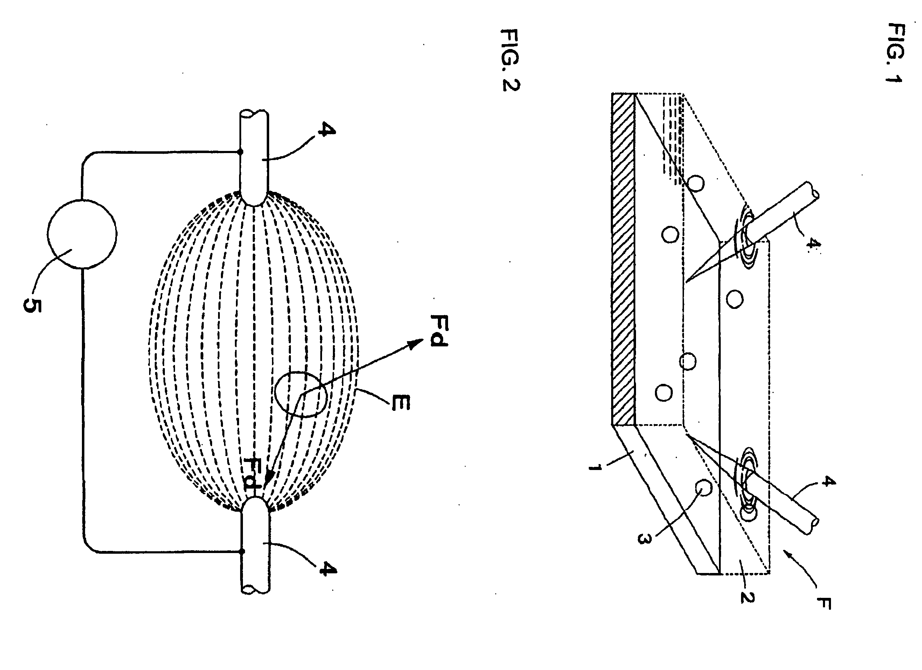

Hereinafter the non-contact manipulation device with an electrode pair and the manipulation methods according to the present invention will be explained with reference to the drawings. Fig. 1 is a schematic diagram showing main components of the non-contact manipulation device with an electrode pair according to the present invention, and is a perspective view of a operation area (F) of the invention.

The operation area (F) is an area to be observed through a microscope or any other means capable of viewing microparticles.

A base (1) is placed on the operation area (F). A suspension liquid layer (2) is placed on the base (1). The suspension liquid layer (2) contains microparticles (3). Hereinafter examples of cells as particles (3) will be described, however, the present invention is applicable to other microparticles (for example, polymer microparticles or liposomes) than cells.

The base (1) may be a bottom of a container holding suspension liquid, or a bottom of any other means holding suspension liquid. Alternatively, using a base of sample holder (1), the suspension liquid layer (2) may be formed on the base (1) by dropping suspension liquid on a base of sample holder (1).

The distal ends of a pair of electrodes (4) are put into the suspension liquid layer (2). The electrode (4) is a needle electrode with the distal end shapened.

The electrodes (4) are connected to a power supply (not shown in Fig. 1). An AC voltage or a DC voltage is applied to the electrodes (4) by the power supply. An application of the voltage creates an electric field, which becomes substantially parallel to the surface of the liquid suspension layer (2).

Fig. 2 is a schematic drawing of the operation area shown in Fig. 1.

When an AC voltage is applied to the pair of electrodes (4) connected to a power supply (5), an inhomogeneous electric field (E) is generated in the space between the pair of electrodes (4). Microparticles (cells) (3) in this electric field (E) are polarized. The polarized microparticle (cell) (3) moves such that the inhomogeneous characteristics of the electric field (E) become homogeneous.

It is desirable that the frequency of the AC voltage applied is 100 MHz or less. This is because it is not possible to effectively trap and move a microparticle (cell) (3) if the frequency exceeds 100 MHz.

In addition, it is desirable that the amplitude of the AC voltage applied is between 5 mV and 100 V, and the distance between the electrodes (the distance between the most distal ends of the needle electrodes) is between 1 $\mu$m and 0.5 mm. If the amplitude is smaller than 5 mV and/or the distance between the electrodes is over 0.5 mm, the generated electric field (E) is too weak to effectively trap and move a microparticle (cell) (3). If the voltage is higher than 100 V and/or the distance between the electrodes is less than 1 $\mu$m, the force applied to a microparticle (cell) by the electric field (E) is so strong that the microparticle (cell) is destroyed.

The force to move a microparticle (cell) (3) "$F_d$" is expressed in the following formula: [

[Formula 1]

$$F_d = 2\pi a^3 Re(\varepsilon_s) Re(K) grad(0.5E^2_m)$$

[Formula 2]

$$K = (\varepsilon_p - \varepsilon_s)/(\varepsilon_p + 2\varepsilon_s)$$

Here, "a" represents the radius of a microparticle (cell) (3) when it is assumed as a sphere. "Re(x)" is the real part of a complex number "x". "$\varepsilon_p$" is the complex dielectric constant of a microparticle (cell) (3), and "$\varepsilon_s$" is the complex dielectric constant of the solvent. In addition, "$E_m$" represents the amplitude of the electric field applied.

The orientation of the above force "$F_d$" varies depending on the frequency of the AC voltage applied. Selection of a frequency enables to move a microparticle (cell) (3) away from the electric field, to attract a microparticles (cell) (3) to either electrodes (4), or to immobilize a microparticles (cell) (3). In addition, the orientation and strength of the acting force vary depending on the type of microparticle (3) or the suspension liquid. Therefore, the frequency has to be adjusted depending on the type of a microparticle or suspension liquid to perform a desired movement.

It is possible to change the velocity of a microparticle (cell) (3) by modulating the amplitude of the AC voltage.

If the diameter of a microparticle (3) is 100 μm or less, it is possible to move the microparticle (3) by electrophoresis. If the microparticle (3) is positively charged, it moves toward the negative electrode, and if the microparticle (3) is negatively charged, it moves toward the positive electrode. If an AC voltage is applied to them, the polarity of the electrodes (4) is switched alternately. As a result, the microparticles (3) are reciprocated between a pair of electrodes (4).

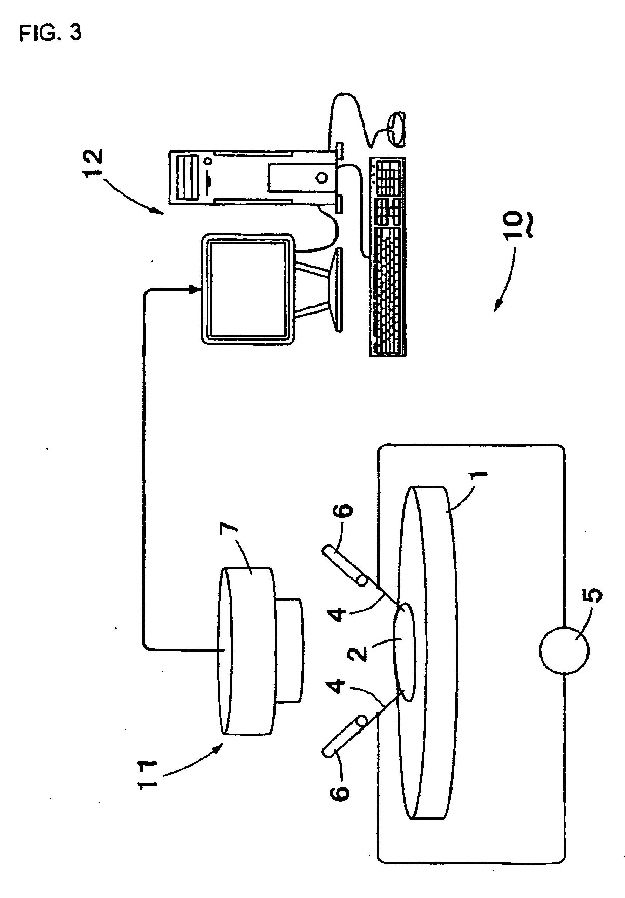

Fig. 3 is an overall schematic diagram of a non-contact manipulation device with an electrode pair according to the present invention. The manipulation device (10) mainly comprises a microscope (11), and a power supply (5), and monitoring device (12).

A stage of a microscope (11) is used as a base (1). A suspension liquid, in which microparticles (3) are suspended, is dropped onto the stage (base) (1) to form a suspension liquid layer (2). A pair of needle electrodes (4) is put in the suspension liquid layer (2). The needle electrodes (4) are electrically connected to the power supply (5) which generates an AC voltage.

It is desirable for the power supply (5) to have functions of adjusting the frequency, amplitude, and waveform of the AC voltage. In addition, it is desirable for the power supply (5) to be connected to an oscilloscope or to be equipped with a waveform display so that the waveform of the AC voltage is monitored. Moreover, it is desirable that DC voltage is applicable.

The needle electrodes (4) are fixed to a pair of operation arms (6) equipped on the microscope (11) respectively. It is possible to move the operation arms (6) up and down, left and right, and back and forth. Moreover, it is also possible to move them independently. It is desirable to move the operation arms (6), for example, by an electric motor, so that the needle electrodes (4) may be positioned precisely. Furthermore, this configuration makes it possible to repeat motions of the operation arm (6) such as rotation or reciprocation.

The suspension liquid layer (2) and at least the distal ends of the needle electrodes (4) are captured by an imaging device (7) located above the base (1). The images taken by the imaging device (7) are displayed on a monitor (12).

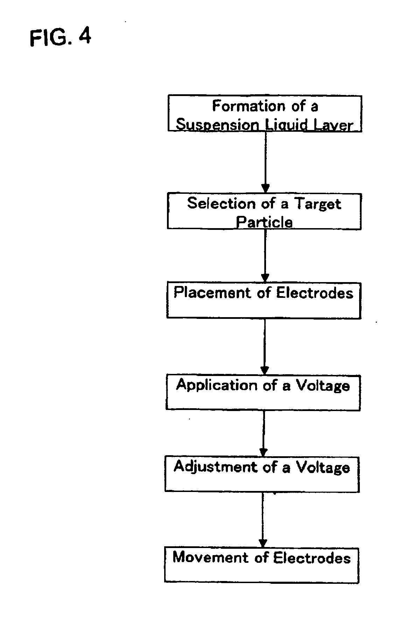

Fig. 4 is a flow diagram indicating the general procedure to move microparticles (3) using the manipulation device (10) shown in Fig. 1 to Fig. 3.

As shown in Fig. 4, firstly, a suspension liquid layer (2) is formed on the base (1). At this step, it is desirable that the concentration of the microparticles (3) in the suspension liquid layer (2) is adjusted. This is because if the concentration of the microparticle is too high, excess microparticles (3) easily get adhered to the needle electrodes (4) put in the suspension liquid layer (2), so that the movement of the microparticle is disturbed. It also becomes difficult to search for a target microparticle (3) if the concentration of the microparticle is too low.

Secondly, a target microparticle (3) is selected. The microparticles (3) displayed on the monitor (12) are observed, and a desired microparticle (3) is selected. For example, if the microparticle (3) is a cell, it is possible to label it by adding a fluorescent color only to a particular cell, so that only a particular cell is fluorescent and then selected as a target cell.

After the target microparticle (3) is selected, the needle electrodes (4) are placed so that the target microparticle (3) stays between the needle electrodes. Here, the needle electrodes (4) are placed without contact with the target microparticle (3).

After the needle electrodes (4) are placed, an AC voltage is applied to the electrodes. Here, it is desirable to increase the voltage gradually. This is because the initial application of high voltage may destroy the cell wall and the membrane, if a microparticle (3) is a cell.

After an AC voltage is applied to the electrodes, it is possible to adjust the frequency of the AC voltage with observing the behavior of the target microparticle (3) on the monitor (12). An exemplified case is explained below. In this case, the target microparticle (3) is moved such that the target microparticle (3) is pushed out of the electric field generated between the needle electrodes (4). If the target microparticle (3) is attracted to one of the needle electrodes (4), the frequency is increased or decreased such that the target cell is pushed out of the field.

Here, the voltage may also be adjusted. For example, if the velocity of the target microparticle (3) is too high, it is possible to reduce the velocity by decreasing the voltage.

After the voltage is adjusted, the needle electrodes (4) are manipulated such that the target microparticle (3) is moved as desired.

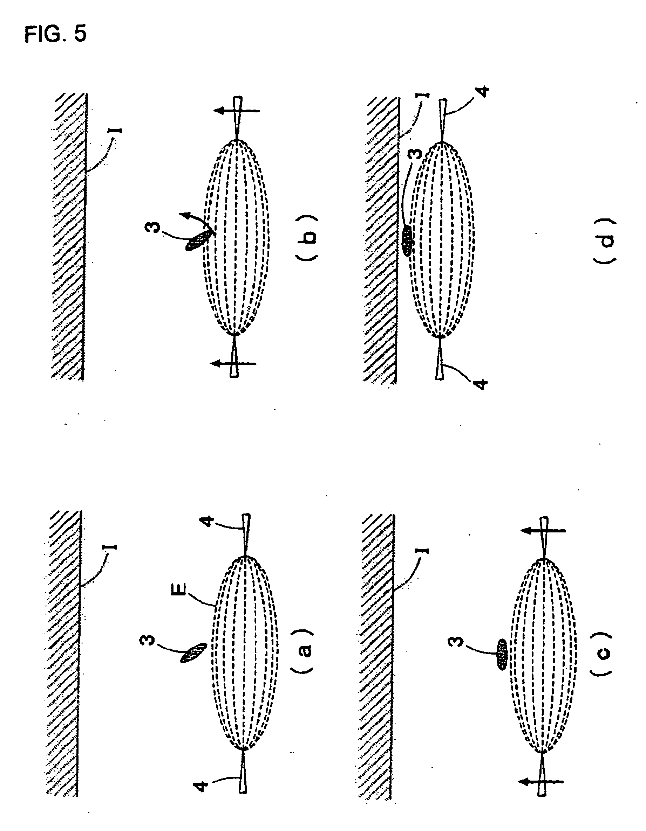

Thirdly, a more detailed example of manipulation to move a microparticle is shown. Fig. 5 shows a specific example of the operation. An ellipsoidal cell is considered as a target microparticle. A cell (3) is placed near the interface with the long axis of the cell (3) parallel to the interface. Figs. 5 (a) to 5 (d) show this process in sequence.

First, a cell (3) is placed with its long axis not parallel to but inclined to the interface (I) (refer to Fig. 5(a)). In addition, an AC voltage is applied to the needle electrodes (4), and the frequency and amplitude of the voltage are adjusted as shown in the step of voltage adjustment in Fig. 4 such that the cell (3) is pushed out of the electric field (E).

If the needle electrodes (4) are moved toward the interface (I), one of the ends of the long axis of the cell (3) enters the electric field (E). (Refer to Fig. 5(b).) The long axis of the cell, with its end in the electric field (E), is pushed out of the electric field (E), and then the cell undergoes rotational movement (in the direction of the arrow in Fig. 5(b)). Next, the needle electrodes (4) are moved toward the interface (I) until the long axis of the cell (3) becomes parallel to the interface (I) (refer to Fig. 5(d)). Since the end of the short axis of the cell (3) is pushed by the electric field (E), it becomes possible to place the cell (3) near the interface (I) with the long axis of the cell (3) parallel to the interface (I) by moving the needle electrodes (4).

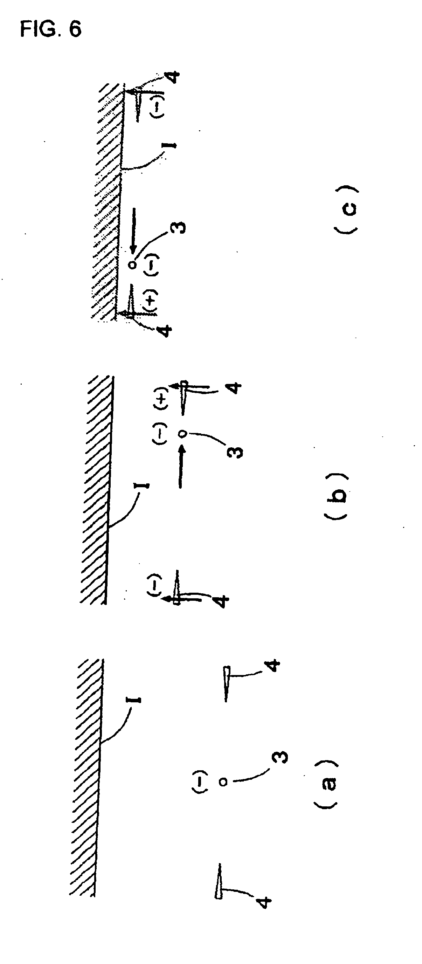

Fig. 6 shows the method to move a microparticle (3) by electrophoresis. The process in Fig. 6 shows a movement of a microparticle (3) to near-interface (I). Fig. 6(a) to Fig. 6(c) show the movement of the microparticle (3) in this process in sequence.

First, as shown in Fig. 6(a), a microparticle (3) is placed between a pair of needle electrodes (4). The microparticle (3) is negatively charged.

Here, if an AC voltage is applied to the needle electrodes (4), the polarity of the voltage of the needle electrodes (4) is switched alternately. As shown in Fig. 6(b), if the voltage of the right-hand needle electrode (4) is positive and the voltage of the left-hand needle electrode (4) is negative, the microparticle (3) is moved toward the right, conversely if the left-hand needle electrode (4) is positively charged, the microparticle (3) is moved toward the left. As such, the microparticle (3) reciprocates left and right between the needle electrodes (4). During the reciprocation of the microparticle (3), if the needle electrodes (4) are moved toward the interface (I), the microparticle (3) approaches the interface (I) with reciprocating left and right.

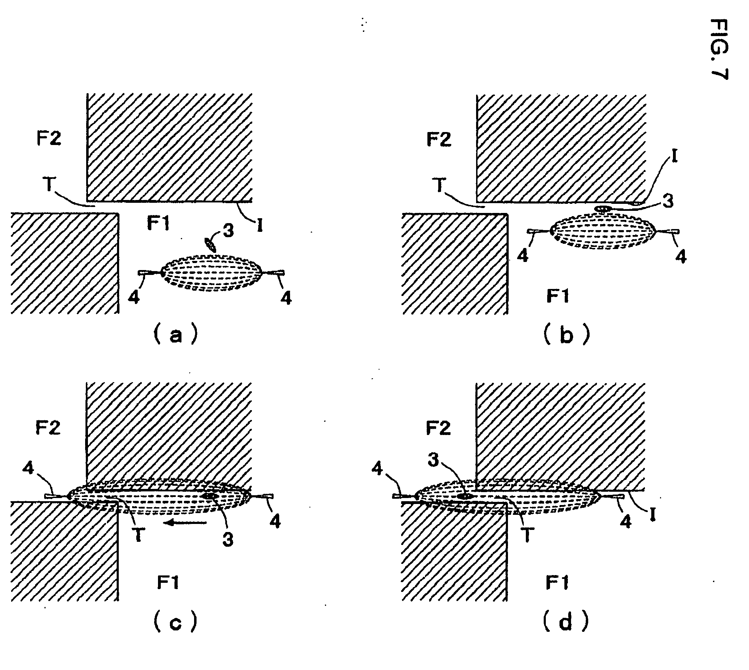

Fig. 7 shows a further alternative embodiment of moving a microparticle shown in Fig. 5. In the example shown in Fig. 7, an ellipsoidal cell (3) is moved from a first operation area (F1) to a second operation area (F2). The first operation area (F1) and the second operation area (F2) are connected through a narrow channel (T). The width of the narrow channel (T) is narrower than the long axis of the cell (3) and wider than the short axis of the cell (3). All the cell (3) exists in the first operation area (F1), but no cell exists in the second operation area (F2).

As shown in Fig. 7(a), first, the cell (3) is not parallel to but inclined to the interface (I) extending from the wall face of the narrow channel (T). Here, according to the moving procedure described in regard to Fig. 5, the cell (3) is placed with its long axis parallel to the interface (I) such that the cell (3) is placed to the near-interface (I) (refer to Fig. 7(b)).

Next, one of the needle electrodes (4) is placed in the second operation area (F2), and the other needle electrode (4) is placed in the first operation area (F1). Here, the line connecting needle electrodes (4) is substantially parallel to the axis of the narrow channel (T).

In this state, the frequency of the voltage is adjusted so that the cell (3) is attracted to the needle electrode (4) in the second operation area (F2). As a result, the cell (3) moves toward the left (refer to Fig. 7(c)). Here, since the long axis of the cell (3) is parallel to the axis of the narrow channel (T), the cell (3) may pass through the narrow channel (T) without having any contact with the walls of the narrow channel (T). In addition, as shown in Fig. 7(d), the left-hand needle electrode (4) may be moved to the left in accordance with the movement of the cell (3). The cell (3) passes through the narrow channel (T), and reaches the second operation area (F2).

As such, it is possible to easily select a desired cell (3), and transfer the selected cell to a particular space without any contact. It is also possible to sort the types of microparticles in a first operation area (F1) and a second operation area (F2).

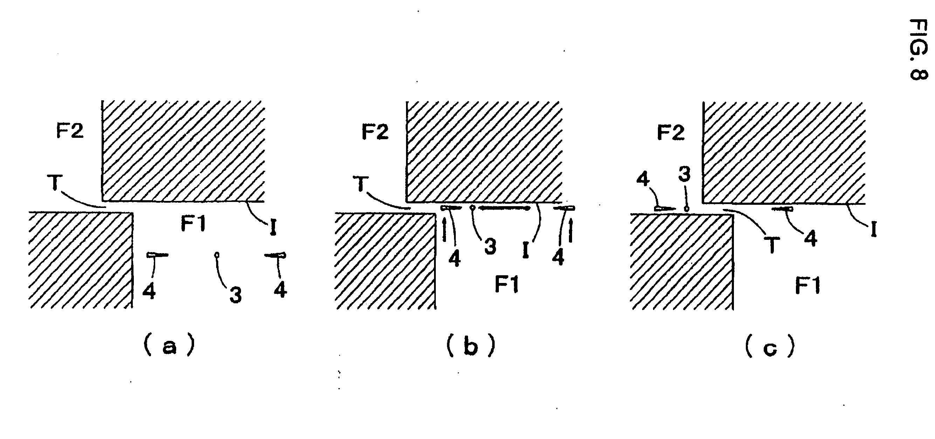

Fig. 8 shows the movement of a microparticle (3) from a first operation area (F1) to a second operation area (F2) by electrophoresis. The configuration of an operation area (F1) and an (F2) are the same as the configuration of Fig. 7 First, a pair of needle electrodes (4) is placed on the left side and the right side of the microparticle (3) in the first operation area (F1) (refer to Fig. 8(a)) Then, in the same way as described with respect to Fig. 6, an AC voltage is applied to the needle electrodes (4), and the needle electrodes (4) are brought close to the interface (I) extending from the wall of the narrow channel (T) (refer to Fig. 8(b)). Here, the microparticle (3) approaches the interface (I), with reciprocating left and right between the needle electrodes (4).

After that, one of the needle electrodes (4) is placed in the second operation area (F2) so that the line connecting the pair of needle electrodes (4) is parallel to the axis of the narrow channel (T), and that the line passes through the narrow channel (T). In this state, by applying a positive voltage to the needle electrode (4) in the second operation area (F2), the negatively-charged microparticle (3) is attracted to the needle electrode (4) in the second operation area (F2), passes through the narrow channel (T), and reaches the second operation area (F2) from the first operation area (F1).

Although in the description of Fig. 5 to Fig. 8 above, the pair of needle electrodes (4) was moving in the same direction at the same velocity for the simplicity, in the actual operation to move microparticles, each needle electrode (4) may be operated independently. This means that the needle electrodes (4) are independently manipulated so that the microparticle is moved toward the desired direction or the axis of the microparticle is directed toward the desired orientation in accordance with the image of the microparticle (3) displayed on the monitor (12).

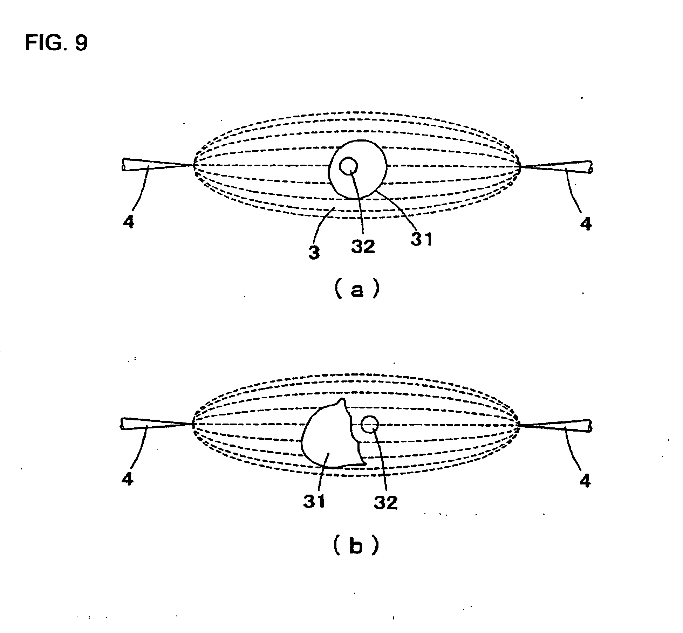

Fig. 9 shows the method to separate and collect the internal organelles of a cell (3).

At the step of voltage application described in regard to Fig. 4, by applying a relatively high voltage to the needle electrodes (4), it becomes possible to destroy the external cellular structures, namely cell walls or cell membranes.

As shown in Fig. 9(a), needle electrodes (4) are placed so that a cell (3) stays between them, and a voltage is applied. By adjusting the voltage, the external cellular structure (31) is destroyed, and a cell organelle (32) (a cell nucleus is shown as the internal organelle) is released out of the external organization of the cell (31) (refer to Fig. 9(b)). Then, it is possible to move the organelle (32) as a target microparticle in the way shown in Figs. 5 to 7.

As an improved device of the above described, it is also possible to coat the portions of the needle electrodes (4) other than the distal ends with an insulating material. Since, by making only the distal ends exposed, it is possible to further limit the accessible range of the electric field, the moving of untargeted microparticles (3) may be avoided.

Operations other than those above described movement of a microparticle are also applicable to the present invention. For example, an alternative embodiment of the operations described regarding Fig. 5 allows fusion of cells aligned on the interface (I), by deterioration of the culture condition in the suspension (for example, by depletion of the nutrients necessary for the cell survival). Another method of cell fusion is also possible. To form a cell population, cells are first aligned with each long axis parallel to the interface (I). Secondly, the needle electrodes are brought close to the ends of the cell population. Thirdly, a DC voltage is applied to the ends of the cell population so that the cells are fused.

Moreover, the present invention may also be applied to move a microscopic region comprising a liquid with a less stable shape than a microparticle or a cell described above.

Instead of the suspension liquid layer (2) in which the microparticles described above are suspended, a layer comprising one type of a liquid (herein called a first liquid) is prepared. Then, another liquid (herein called a second liquid) is dropped into this liquid. The first and second liquids have a different dielectric constant from each other.

When the second liquid is dropped, a microscopic region filled with the second liquid is formed in the liquid layer. Here, the frequency of the AC voltage applied to needle electrodes (4) is set so that an electric field (E) acts on the second liquid. Then, the microscopic region of the second liquid to be moved is selected, and the needle electrodes (4) are placed such that this microscopic region stays between the needle electrodes without any contact. Then, an AC voltage is applied, and the needle electrodes (4) are moved.

As such, since the present invention is able to move a microscopic region comprising a liquid, it is also possible, for example, to transfer microparticles coated with oil droplets.

Although methods are described above where movement is performed without having a target microparticle or a target cell in contact with needle electrodes (4), it is also possible to provide novel operations by making these targets into contact with needle electrodes (4).

Although the rotation of the cell described in regard to Fig. 5 and Fig. 6 involves rotation of an anisotropic microobject, Fig. 10 shows a rotational operation of a spherical body (3) (a cell, a liposome, or a polymer microparticle). Fig. 10 is a drawing of a cross-sectional view of the suspension liquid layer.

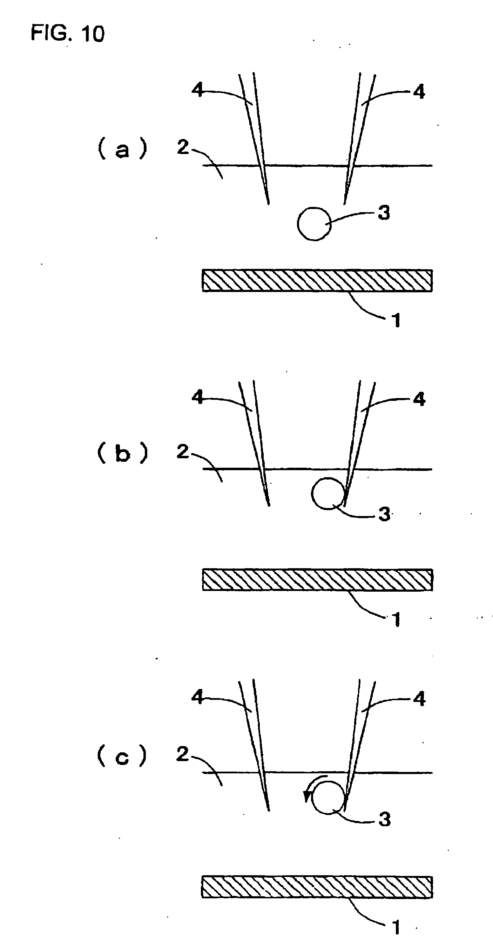

In this operation, a pair of needle electrodes (4) is put close to a spherical body (3) in the suspension liquid layer (2) (refer to Fig. 10(a)). Then, the frequency of the AC voltage between the needle electrodes (4) and the position of the needle electrodes (4) are adjusted so that the spherical body (3) moves toward one of the needle electrodes (4) (in Fig. 10, the right-hand needle electrode (4)) to move the spherical body (3). Then, the spherical body (3) is brought in contacted with the needle electrode (4) (refer to Fig. 10 (b)). In this state, a constant adhesive force is generated between the outer wall of the spherical body (3) and the needle electrode (4).

After that, the frequency of the AC voltage is set such that a dielectrophoretic force "$F_d$" acts to move the spherical body (3) away from this needle electrode (4). Here, the frequency and amplitude of the AC voltage are set such that the dielectrophoretic force "$F_d$" does not exceed the adhesive force described above.

In the situation where the spherical body (3) is not able to move away from the needle electrode (4), the dielectrophoretic force "$F_d$" acts as a rotational moment to the spherical body (3), so that the spherical body (3) rotates on the needle electrode (4).

Here, it is possible to adjust revolutions by changing the frequency and amplitude of the AC voltage.

It is also possible to rotate the spherical body (3) by placing the needle electrodes (4) close to the spherical body (3) without any contact with the spherical body (3), and by performing the same operation described above.

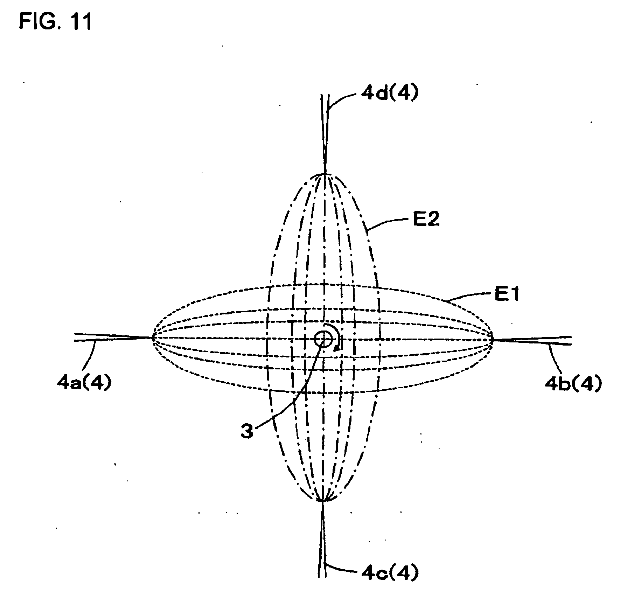

Fig. 11 is a further application of the present invention.

Although the methods to move or rotate objects such as microparticles or cells (3) using a pair of electrodes (4) are described above, two or more pairs of electrodes (4) may be used. Fig. 11 shows a method to employ two pairs of electrodes (4).

The electrodes (4) in two pairs are evenly placed to surround the microparticle (3). An electric field (E1) is generated between one of the two pairs of electrodes (4a and 4b), and another electric field (E2) is generated between the other pair of electrodes (4c and 4d). The intersection of these electric fields (E1, E2) provides more precise control of, for example, the rotation of a microparticle (3).

For instance, in the example shown in Fig. 11, the frequency and amplitude of the AC voltage applied to one pair of electrodes (4a and 4b) and the other pair (4c and 4d) are set to the same value. Then, the phase difference between one pair of electrodes (4a and 4b) and the other pair of electrodes (4c and 4d) are set at 90 degrees. As a result, the applied electric fields rotate in the same manner as the conventional rotational electric field method, and a torque is accordingly acted on the microparticle (3), so that the microparticle (3) is rotated (for example, refer to M. P. Hughes, "Nanoelectromechanics in Engineering and Biology" (CRC Press, 2003)). This enables adjustment of rotational velocity of a microparticle (3) with high level of precision by setting a frequency and amplitude.

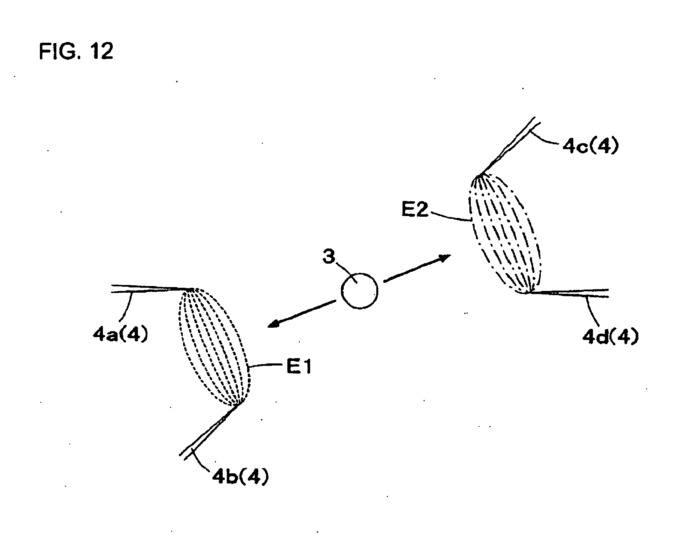

Fig. 12 shows another example in which two pairs of needle electrodes (4) are used.

Although the example shown in Fig. 12 is almost the same as the example shown in Fig. 11, a first electrode (4a) is placed next to a second electrode (4b), and a third electrode (4c) is placed next to a fourth electrode (4d). In addition, an electric field (E1) is generated between the first pair of electrodes (4a and 4b), and a second electric field (E2) is generated between the second pair of electrodes (4c and 4d).

The frequency and amplitude of the AC voltage applied to the first pair of electrodes (4a and 4b) and the second pair of electrodes (4c and 4d) are adjusted such that the microparticle (3) moves away from the needle electrodes (4). This allows us, for example, to make the microparticle (3) reciprocate between the distal ends of the needle electrodes (4).

When a pair of electrodes (4) was used, the input parameters for the movement of a microparticle (3) were the position and moving direction of the electrodes (4), amplitude of the AC voltage or magnitude of the DC voltage applied to the electrodes (4), and the frequency of the AC voltage applied to the electrodes (4). Using multi-pairs of electrodes (4), however, allows the use of an angle formed between electric fields (E1 and E2) and a phase difference in AC voltages applied to the space between pairs of electrodes (4) as input parameters for the movement of the microparticle (3) in addition to the input parameters above. For example, it is possible to vary or to adjust the angle formed by the electric fields (E1 and E2) at the step of moving electrodes shown in Fig. 4. It is also possible to vary and adjust the phase difference of AC voltages applied between the pairs of electrodes (4) at the step of adjusting the voltage shown in Fig. 4. In this way, it is possible to give more complicated movement to the microparticle (3), so that desired motion control may be achieved.

The present invention is preferably applied to a device and a method which may efficiently perform operations such as the moving and sorting of microparticles, cell fusion, or the collection of cellular organelles.

BRIEF DESCRIPTION OF THE DRAWINGS

Fig. 1 is a drawing to show main components of a manipulation device according to the present invention.
Fig. 2 is a schematic representation of the main components in Fig. 1.
Fig. 3 is a schematic drawing of the overall manipulation device according to the present invention.
Fig. 4 is a flow diagram of a manipulation method according to the present invention.
Fig. 5 is a drawing to show one example of manipulation methods according to the present invention.
Fig. 6 is a drawing to show one example of manipulation methods according to the present invention.
Fig. 7 is a drawing to show one example of manipulation methods according to the present invention.
Fig. 8 is a drawing to show one example of manipulation methods according to the present invention.
Fig. 9 is a drawing to show one example of manipulation methods according to the present invention.
Fig. 10 is a drawing to show one example of manipulation methods according to the present invention.
Fig. 11 is a drawing to show one example of manipulation methods according to the present invention. In the example of Fig. 11, two pairs of electrodes are used.
Fig. 12 is a drawing to show one example of manipulation methods according to the present invention. In the example of Fig. 11, two pairs of electrodes are used.

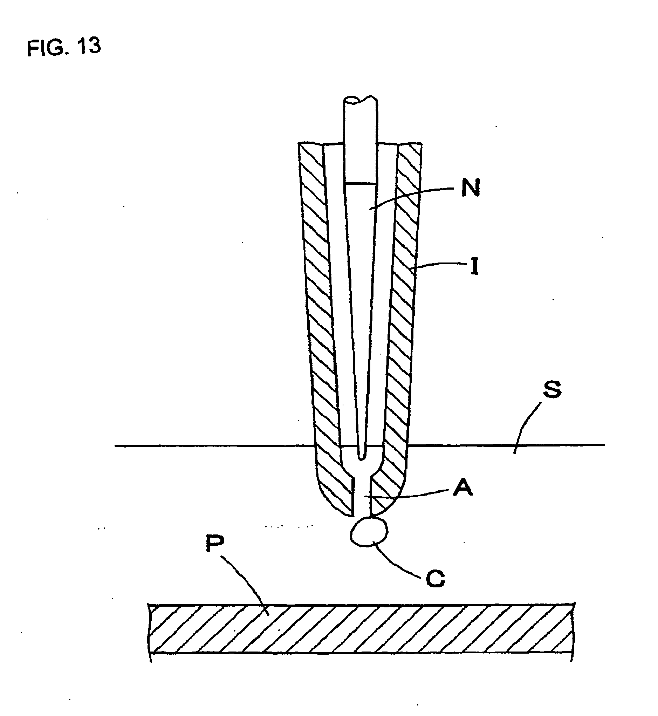

Fig. 13 is a drawing to show a manipulation device of the conventional invention.

Explanation of symbols

1      a base
10     a manipulation device
11     a microscope
2      a suspension liquid layer
3      a microparticle
4      an electorode
5      a power supply
F1     a first operation area
F2     a second operation area
T      a narrow channel

**Claims**

1.  A non-contact manipulation device with an electrode pair comprising:

    a base (1);
    a suspension liquid layer (2) disposed on the base (1) and containing microparticles(3);
    at least a pair of needle electrodes (4) having the distal ends put into the suspension liquid layer (2); and
    a power supply (5) for supplying an AC voltage to the needle electrodes,
    wherein the at least a pair of needle electrodes (4) configured to move together or independently of each other, and
    wherein an electric field is generated between ends of the at least a pair of needle electrodes (4).

2.  A non-contact manipulation device with an electrode pair comprising:

    a base (1);
    a suspension liquid layer (2) disposed on the base (1);
    at least a pair of needle electrodes (4) having the distal ends put into the suspension liquid layer (2); and
    a power supply (5) for supplying an AC voltage to the needle electrodes (4),
    wherein the at least a pair of needle electrodes (4) is configured to move together or independently of each other,
    the suspension liquid layer (2) comprises a first liquid and a second liquid having a dielectric constant different from the first liquid;
    the second liquid occupies a microscopic region of the suspension liquid layer;
    an electric field is generated between the ends of the at least a pair of needle electrodes.

3.  The non-contact manipulation device with an electrode pair according to one of claims 1 or 2, wherein the at least a pair of needle electrodes (4) is configured to move independently of the base (1).

4.  The non-contact manipulation device with an electrode pair according to one of claims 1 to 3, wherein the power supply (5) is configured to modulate the frequency of the AC voltage.

5.  The non-contact manipulation device with an electrode pair according to one of claims 1 to 4, wherein the power supply (5) is configured to modulate the amplitude of the AC voltage.

6.  The non-contact manipulation device with an electrode pair according to one of claims 1 to 5, wherein the power supply (5) is configured to modulate the waveform of the AC voltage.

7.  The non-contact-manipulation device with an electrode pair according to one of claims 1 to 2, wherein the power supply (5) is configured to apply optionally a DC voltage.

8.  The non-contact manipulation device with an electrode pair according to one of claims 1 to 7, wherein the base (1) is placed in observable area through a microscope (11) to allow the microparticles (3) to be observed.

9.  The non-contact manipulation device with an electrode pair according to one of claims 1 to 6, wherein the at least a pair of needle electrodes (4) is multi-pairs of needle electrodes, and a phase difference of the AC voltage between one pair of needle electrodes (4) and the other pair of needle electrodes (4) is variable.

10. A manipulation method of the non-contact manipulation device comprising:

    selecting a microparticle (3) in the suspension liquid layer (2);
    placing at least a pair of needle electrodes such that the selected microperticle (3) is placed between the needle electrodes (4) without any contact with the needle electrodes (4);
    applying voltage to the needle electrodes (4);
    adjusting the frequency of the voltage; and
    moving the needle electrodes (4) such that the at least a pair of needle electrodes (4) is configured to move together or independently of each other.

11. The non-contact manipulation method with an electrode pair (4) according to claim 10, wherein the suspension liquid layer (2) is placed on a first and a second operation area (F1, F2), which are connected through a narrow channel (T).

12. A non-contact manipulation method with an electrode pair according to claim 11, wherein the microparticle (3) is ellipsoidal, and the width of the narrow channel (T) is narrower than a long axis of the particle (3) and wider than a short axis of the particle (3).

13. A non-contact manipulation method with an electrode pair according to one of claims 10 to 12, wherein the at least a pair of needle electrodes (4) is multi-pairs of needle electrodes (4), and the step of adjusting the frequency of the voltage further comprising modification of a phase difference of the AC voltage between one pair of needle electrodes (4) and the other pair of needle electrodes (4).

14. A non-contact manipulation method with an electrode pair according to one of claims 10 to 13, wherein the at least a pair of needle electrodes (4) is multi-pairs of needle electrodes (4), and the step of moving the needle electrodes (4) further comprising modification of an angle formed between an electric field created by one pair of needle electrodes (4) and an electric field created by the other pair of needle electrodes (4) of the multi pairs of needle electrodes (4).

15. A non-contact manipulation method with an electrode pair according to one of claims 10 to 14, wherein the microparticle (3) is a cell, and the step of moving the needle electrodes (4) comprises depletion of the nutrients necessary for the cell survival in the suspension liquid layer (2) after placing the cell next to other cells.

16. A non-contact manipulation method with an electrode pair according to one of claims 10 to 15, wherein the microparticle (3) is a cell, and the step of moving the needle electrodes (4) comprises,
placing the cell to form a cell population which is made up with cells lined next to each other, and
applying a DC voltage to the distals of the cell population.

17. A non-contact manipulation method with an electrode pair according to claim 15 or 16, wherein the step of moving the needle electrodes (4) comprising adjustment of the cell orientation.

18. A non-contact manipulation method with an electrode pair comprising:

selecting a cell in the suspension liquid layer (2);
placing at least one pair of needle electrodes (4) such that the selected cell is placed between the needle electrodes (4) and is not in contact with the cell;
applying voltage to the needle electrodes (4), to destroy external structures of the cell, and to release internal organelles of the cell;
applying the voltage to the needle electrodes (4);
adjusting the frequency of the voltage; and
moving the needle electrodes (4),
wherein the at least a pair of needle electrodes (4) is configured to move together or independently of each other.

19. A non-contact manipulation method with an electrode pair comprising:

selecting a cell in the suspension liquid layer (2);
dropping a second liquid into a liquid layer (2) comprised of the first liquid, the second liquid having a dielectric constant different from the first liquid;
selecting one of areas formed in the liquid layer (2) which is comprised of the second liquid;
placing at least one pair of needle electrodes (4) such that the selected area is placed between the needle electrodes (4) and not to touch the area;
applying voltage to the needle electrodes (4);
adjusting the frequency of the voltage; and
moving the needle electrodes (4),
wherein the at least a pair of needle electrodes(4) is configured to move together or independently of each other.

**Patentansprüche**

1. Berührungsfreie Manipulationsvorrichtung mit einem Elektrodenpaar, umfassend:

eine Basis (1);

eine Suspensionsflüssigkeitsschicht (2), die auf der Basis (1) angeordnet ist und Mikropartikel (3) enthält;

wenigstens ein Paar von Nadelelektroden (4), deren distale Enden in die Suspensionsflüssigkeitsschicht (2) getaucht sind; und

eine Spannungsversorgung (5) zum Liefern einer Wechselspannung an die Nadelelektroden,

wobei das wenigstens eine Paar von Nadelelektroden (4) dafür konfiguriert ist, sich gemeinsam oder unabhängig voneinander zu bewegen, und

wobei ein elektrisches Feld zwischen Enden des wenigstens einen Paars von Nadelelektroden (4) erzeugt wird.

2. Berührungsfreie Manipulationsvorrichtung mit einem Elektrodenpaar, umfassend:

eine Basis (1);

eine Suspensionsflüssigkeitsschicht (2), die auf der Basis (1) angeordnet ist;

wenigstens ein Paar von Nadelelektroden (4), deren distale Enden in die Suspensionsflüssigkeitsschicht (2) getaucht sind; und

eine Spannungsversorgung (5) zum Liefern einer Wechselspannung an die Nadelelektroden (4),

wobei das wenigstens eine Paar von Nadelelektroden (4) dafür konfiguriert ist, sich gemeinsam oder unabhängig voneinander zu bewegen,

die Suspensionsflüssigkeitsschicht (2) eine erste Flüssigkeit und eine zweite Flüssigkeit, die eine andere Dielektrizitätskonstante als die erste Flüssigkeit aufweist, umfasst;

die zweite Flüssigkeit einen mikroskopischen Bereich der Suspensionsflüssigkeitsschicht einnimmt;

ein elektrisches Feld zwischen den Enden des wenigstens einen Paars von Nadelelektroden erzeugt wird.

3. Berührungsfreie Manipulationsvorrichtung mit einem Elektrodenpaar nach einem der Ansprüche 1 oder 2, wobei das wenigstens eine Paar von Nadelelektroden (4) dafür konfiguriert ist, sich unabhängig von der Basis (1) zu bewegen.

4. Berührungsfreie Manipulationsvorrichtung mit einem Elektrodenpaar nach einem der Ansprüche 1 bis 3, wobei die Spannungsversorgung (5) dafür konfiguriert ist, die Frequenz der Wechselspannung zu modulieren.

5. Berührungsfreie Manipulationsvorrichtung mit einem Elektrodenpaar nach einem der Ansprüche 1 bis 4, wobei die Spannungsversorgung (5) dafür konfiguriert ist, die Amplitude der Wechselspannung zu modulieren.

6. Berührungsfreie Manipulationsvorrichtung mit einem Elektrodenpaar nach einem der Ansprüche 1 bis 5, wobei die Spannungsversorgung (5) dafür konfiguriert ist, die Wellenform der Wechselspannung zu modulieren.

7. Berührungsfreie Manipulationsvorrichtung mit einem Elektrodenpaar nach einem der Ansprüche 1 bis 2, wobei die Spannungsversorgung (5) dafür konfiguriert ist, wahlweise eine Gleichspannung anzulegen.

8. Berührungsfreie Manipulationsvorrichtung mit einem Elektrodenpaar nach einem der Ansprüche 1 bis 7, wobei die Basis (1) in einem durch ein Mikroskop (11) beobachtbaren Bereich angeordnet ist, um die Beobachtung der Mikropartikel (3) zu ermöglichen.

9. Berührungsfreie Manipulationsvorrichtung mit einem Elektrodenpaar nach einem der Ansprüche 1 bis 6, wobei es sich bei dem wenigstens einen Paar von Nadelelektroden (4) um Multipaare von Nadelelektroden handelt und eine Phasendifferenz der Wechselspannung zwischen einem Paar von Nadelelektroden (4) und dem anderen Paar von Nadelelektroden (4) variabel ist.

10. Manipulationsverfahren der berührungsfreien Manipulationsvorrichtung, umfassend:

das Auswählen einer Mikropartikel (3) in der Suspensionsflüssigkeitsschicht (2);

das Anordnen wenigstens eines Paars von Nadelelektroden, derart, dass die ausgewählte Mikropartikel (3) ohne jeglichen Kontakt mit den Nadelelektroden (4) zwischen den Nadelelektroden (4) angeordnet ist;

das Anlegen einer Spannung an die Nadelelektroden (4);

das Einstellen der Frequenz der Spannung; und

das Bewegen der Nadelelektroden (4), derart, dass das wenigstens eine Paar von Nadelelektroden (4) so konfiguriert ist, dass es sich gemeinsam oder unabhängig voneinander bewegt.

**11.** Berührungsfreies Manipulationsverfahren mit einem Elektrodenpaar (4) nach Anspruch 10, wobei die Suspensionsflüssigkeitsschicht (2) auf einem ersten und einem zweiten Arbeitsbereich (F1, F2) angeordnet wird, die durch einen engen Kanal (T) verbunden sind.

**12.** Berührungsfreies Manipulationsverfahren mit einem Elektrodenpaar nach Anspruch 11, wobei die Mikropartikel (3) ellipsoidisch ist und die Breite des engen Kanals (T) schmaler als eine große Achse der Partikel (3) und breiter als eine kleine Achse der Partikel (3) ist.

**13.** Berührungsfreies Manipulationsverfahren mit einem Elektrodenpaar nach einem der Ansprüche 10 bis 12, wobei es sich bei dem wenigstens einen Paar von Nadelelektroden (4) um Multipaare von Nadelelektroden (4) handelt und der Schritt des Einstellens der Frequenz der Spannung außerdem die Modifikation einer Phasendifferenz der Wechselspannung zwischen einem Paar von Nadelelektroden (4) und dem anderen Paar von Nadelelektroden (4) umfasst.

**14.** Berührungsfreies Manipulationsverfahren mit einem Elektrodenpaar nach einem der Ansprüche 10 bis 13, wobei es sich bei dem wenigstens einen Paar von Nadelelektroden (4) um Multipaare von Nadelelektroden (4) handelt und der Schritt des Bewegens der Nadelelektroden (4) außerdem die Modifikation eines Winkels umfasst, der zwischen einem elektrischen Feld, das von einem Paar von Nadelelektroden (4) erzeugt wird, und einem elektrischen Feld, das von dem anderen Paar von Nadelelektroden (4) der Multipaare von Nadelelektroden (4) erzeugt wird, gebildet wird.

**15.** Berührungsfreies Manipulationsverfahren mit einem Elektrodenpaar nach einem der Ansprüche 10 bis 14, wobei die Mikropartikel (3) eine Zelle ist und der Schritt des Bewegens der Nadelelektroden (4) nach dem Anordnen der Zelle neben anderen Zellen einen Entzug der Nährstoffe, die für das Überleben der Zellen in der Suspensionsflüssigkeitsschicht (2) erforderlich sind, umfasst.

**16.** Berührungsfreies Manipulationsverfahren mit einem Elektrodenpaar nach einem der Ansprüche 10 bis 15, wobei die Mikropartikel (3) eine Zelle ist und der Schritt des Bewegens der Nadelelektroden (4) das Anordnen der Zelle zur Bildung einer Zellpopulation, die aus Zellen besteht, die nebeneinander aufgereiht sind, und das Anlegen einer Wechselspannung an die Distalen der Zellpopulation umfasst.

**17.** Berührungsfreies Manipulationsverfahren mit einem Elektrodenpaar nach Anspruch 15 oder 16, wobei der Schritt des Bewegens der Nadelelektroden (4) das Einstellen der Zellorientierung umfasst.

**18.** Berührungsfreies Manipulationsverfahren mit einem Elektrodenpaar, umfassend:

das Auswählen einer Zelle in der Suspensionsflüssigkeitsschicht (2);
das Anordnen wenigstens eines Paars von Nadelelektroden (4), derart, dass die ausgewählte Zelle zwischen den Nadelelektroden (4) angeordnet ist und nicht mit der Zelle in Kontakt ist;
das Anlegen einer Spannung an die Nadelelektroden (4), um äußere Strukturen der Zelle zu zerstören und die inneren Organellen der Zelle freizusetzen;
das Anlegen der Spannung an die Nadelelektroden (4);
das Einstellen der Frequenz der Spannung; und
das Bewegen der Nadelelektroden (4),
wobei das wenigstens eine Paar von Nadelelektroden (4) dafür konfiguriert ist, sich gemeinsam oder unabhängig voneinander zu bewegen.

**19.** Berührungsfreies Manipulationsverfahren mit einem Elektrodenpaar, umfassend:

das Auswählen einer Zelle in der Suspensionsflüssigkeitsschicht (2);
das Eintropfen einer zweiten Flüssigkeit in eine Flüssigkeitsschicht (2), die aus der ersten Flüssigkeit besteht, wobei die zweite Flüssigkeit eine andere Dielektrizitätskonstante als die erste Flüssigkeit aufweist;
das Auswählen eines der in der Flüssigkeitsschicht (2) gebildeten Bereiche, der aus der zweiten Flüssigkeit besteht;
das Anordnen wenigstens eines Paars von Nadelelektroden (4), derart, dass der ausgewählte Bereich zwischen den Nadelelektroden (4) angeordnet ist und der Bereich nicht berührt wird;
das Anlegen einer Spannung an die Nadelelektroden (4);
das Einstellen der Frequenz der Spannung; und

das Bewegen der Nadelelektroden (4),
wobei das wenigstens eine Paar von Nadelelektroden (4) dafür konfiguriert ist, sich gemeinsam oder unabhängig voneinander zu bewegen.

**Revendications**

1. Dispositif de manipulation sans contact avec une paire d'électrodes comprenant :

   - une base (1),
   - une couche de suspension liquide (2) sur la base (1) et contenant des microparticules (3),
   - au moins une paire d'électrodes-aiguilles (4) dont les extrémités distales pénètrent dans la couche de suspension liquide (2), et
   - une alimentation (5) pour fournir une tension alternative aux électrodes-aiguilles,
   dans lequel
   - au moins une paire d'électrodes-aiguilles (4) est configurée pour se déplacer en même temps ou indépendamment l'une de l'autre, et
   - un champ électrique est généré entre les extrémités au niveau d'au moins une paire d'électrodes-aiguilles (4).

2. Dispositif de manipulation sans contact avec une paire d'électrodes comprenant :

   - une base (1),
   - une couche de suspension liquide (2) placée sur la base (1),
   - au moins une paire d'électrodes-aiguilles (4) dont l'extrémité distale vient dans la couche de liquide en suspension (2), et
   - une alimentation (5) pour fournir une tension alternative aux électrodes-aiguilles (4),
   - au moins une paire d'électrodes-aiguilles (4) étant configurée pour se déplacer simultanément ou indépendamment l'une de l'autre,
   - la couche de suspension liquide (2) contient un premier liquide et un second liquide, ce dernier ayant une constante diélectrique différente de celle du premier liquide,
   - le second liquide occupant une région microscopique de la couche liquide en suspension, et
   - un champ électrique est formé entre les extrémités d'au moins une paire d'électrodes-aiguilles.

3. Dispositif de manipulation sans contact avec une paire d'électrodes selon la revendication 1 ou 2, dans lequel
   au moins une paire d'électrodes-aiguilles (4) est configurée pour se déplacer indépendamment de la base (1).

4. Dispositif de manipulation sans contact avec une paire d'électrodes selon l'une des revendications 1 à 3, dans lequel
   l'alimentation électrique (5) est configurée pour moduler la fréquence sur la tension alternative.

5. Dispositif de manipulation sans contact avec une paire d'électrodes selon l'une des revendications 1 à 4, dans lequel
   l'alimentation électrique (5) est configurée pour moduler l'amplitude de la tension alternative.

6. Dispositif de manipulation sans contact avec une paire d'électrodes selon l'une des revendications 1 à 5, dans lequel
   l'alimentation électrique (5) est configurée pour moduler la forme ondulée de la tension alternative.

7. Dispositif de manipulation sans contact avec une paire d'électrodes selon la revendication 1 ou 2, dans lequel
   l'alimentation électrique (5) est configurée pour appliquer en option une tension continue.

8. Dispositif de manipulation sans contact avec une paire d'électrodes selon l'une des revendications 1 à 7, dans lequel
   la base (1) est placée dans une zone d'observation d'un microscope (11) pour permettre l'observation des microparticules (3).

**9.** Dispositif de manipulation sans contact avec une paire d'électrodes selon l'une des revendications 1 à 6, dans lequel

au moins une paire d'électrodes-aiguilles (4) est une paire multiple d'électrodes-aiguilles, et la différence de phase de la tension alternative entre une paire d'électrodes sélectionnée (4) et l'autre paire d'électrodes-aiguilles (4), est variable.

**10.** Procédé d'actionnement du dispositif de manipulation sans contact, comprenant les étapes suivantes :

- sélection d'une microparticule (3) de la couche de suspension liquide (2),
- placement d'au moins une paire d'électrodes-aiguilles de façon que la microparticule choisie (3) se place entre les électrodes-aiguilles (4) sans aucun contact avec l'électrode-aiguille (4),
- application de la tension aux électrodes-aiguilles (4),
- ajustage de la fréquence de la tension, et
- déplacement des électrodes-aiguilles (4) de façon qu'au moins une paire d'électrodes-aiguilles (4) soit configurée pour se déplacer ensemble ou indépendamment l'une de l'autre.

**11.** Procédé de manipulation sans contact avec une paire d'électrodes (4) selon la revendication 10, dans lequel

la couche de suspension liquide (2) est placée sur la première et sur la seconde zone de traitement (F1, F2) reliées par l'intermédiaire d'un canal étroit (T).

**12.** Procédé de manipulation sans contact avec une paire d'électrodes selon la revendication 11, dans lequel

la microparticule (3) a une forme ellipsoïdale et la largeur du canal étroit (T) est inférieure au grand axe de la particule (3) et plus large que le petit axe de la particule (3).

**13.** Procédé de manipulation sans contact avec une paire d'électrodes selon l'une des revendications 10 à 12, dans lequel

au moins une paire d'électrodes-aiguilles (4) est une paire multiple d'électrodes-aiguilles (4) et l'étape de réglage de la fréquence de la tension, comprend en outre la modification de la différence de phase de la tension AC déplacée entre une paire d'aiguilles formant des électrodes (4) et l'autre paire d'électrodes-aiguilles (4).

**14.** Procédé de manipulation sans contact avec une paire d'électrodes selon l'une des revendications 10 à 13, dans lequel

au moins une paire d'électrodes-aiguilles (4) est une paire multiple d'électrodes-aiguilles (4) et l'étape de déplacement des électrodes-aiguilles (4) comprend en outre la modification de l'angle formé entre le champ électrique créé par une paire d'électrodes-aiguilles (4) et le champ électrique créé par l'autre paire d'électrodes-aiguilles (4) de la paire multiple d'électrodes-aiguilles (4).

**15.** Procédé de manipulation sans contact avec une paire d'électrodes selon l'une des revendications 10 à 14, dans lequel

la microparticule (3) est une cellule, et l'étape de déplacement des électrodes-aiguilles (4), consiste à appauvrir les agents nutritifs nécessaires à la survie de la cellule dans la couche de suspension liquide (2) après avoir placé la cellule près des autres cellules.

**16.** Procédé de manipulation sans contact avec une paire d'électrodes selon l'une des revendications 10 à 15, dans lequel

la microparticule (3) est une cellule, et l'étape de déplacement des électrodes-aiguilles (4), consiste à placer la cellule pour former une population de cellules, composée de cellules alignées l'une à proximité de l'autre, et appliquer une tension continue aux extrémités distales de la population d'électrodes.

**17.** Procédé de manipulation sans contact avec une paire d'électrodes selon la revendication 15 ou 16, dans lequel

l'étape de déplacement des électrodes-aiguilles (4) consiste à régler l'orientation des cellules.

**18.** Procédé de manipulation sans contact avec une paire d'électrodes comprenant les étapes suivantes :

- sélection d'une cellule dans la couche de suspension liquide (2),

- placer au moins une paire d'électrodes-aiguilles (4) de façon que la cellule choisie se place entre les électrodes-aiguilles (4) et ne soit pas en contact avec la cellule,
- application de la tension des électrodes-aiguilles (4) pour détruire les structures externes de la cellule et libérer les organelles internes de la cellule,
- appliquer la tension aux électrodes de la cellule (4),
- ajuster la fréquence de la tension, et
- déplacer les électrodes-aiguilles (4) de façon qu'au moins une paire d'électrodes-aiguilles (4) soit configurée pour se déplacer ensemble ou indépendamment l'une de l'autre.

**19.** Procédé de manipulation sans contact avec une paire d'électrodes comprenant les étapes suivantes :

- sélection d'une cellule dans la couche de suspension liquide (2),
- laisser tomber un second liquide dans une couche liquide (2) comportant le premier liquide et le second liquide avec une constante diélectrique différente du premier liquide,
- choisir l'une des zones formées dans la couche de liquide (2) comprenant le second liquide,
- placer au moins une paire d'électrodes-aiguilles (4) de façon que la zone sélectionnée se trouve entre les électrodes-aiguilles (4) et ne touche pas la zone,
- application d'une tension aux électrodes-aiguilles (4),
- réglage de la fréquence de la tension, et
- déplacement des électrodes-aiguilles (4),
selon lequel
au moins une paire d'électrodes-aiguilles (4) est configurée pour se déplacer ensemble ou indépendamment l'une de l'autre.

FIG. 1

FIG. 2

FIG. 3

EP 1 854 870 B1

## FIG. 4

```
┌─────────────────────────┐
│      Formation of a      │
│ Suspension Liquid Layer  │
└─────────────────────────┘
             │
             ▼
┌─────────────────────────┐
│    Selection of a Target │
│         Particle         │
└─────────────────────────┘
             │
             ▼
┌─────────────────────────┐
│  Placement of Electrodes │
└─────────────────────────┘
             │
             ▼
┌─────────────────────────┐
│  Application of a Voltage│
└─────────────────────────┘
             │
             ▼
┌─────────────────────────┐
│  Adjustment of a Voltage │
└─────────────────────────┘
             │
             ▼
┌─────────────────────────┐
│  Movement of Electrodes  │
└─────────────────────────┘
```

# FIG. 5

FIG. 6

EP 1 854 870 B1

FIG. 7

FIG. 8

FIG. 9

( a )

( b )

FIG. 10

( a )

4    4

2

3

1

( b )

4    4

2

3

1

( c )

4    4

2

3

1

FIG. 11

FIG. 12

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP H5137576 B

- WO 03104448 A

**Non-patent literature cited in the description**

- **M. P. Hughes.** Nanoelectromechanics in Engineering and Biology. CRC Press, 2003